# EUROPÄISCHE PATENTSCHRIFT

(11) **EP 3 307 869 B1**
(45) Veröffentlichungstag und Bekanntmachung des Hinweises auf die Patenterteilung: **29.07.2020**
(21) Anmeldenummer: 16732247.8
(22) Anmeldetag: 08.06.2016
(51) Int. Cl.: C12M 3/00, C12M 1/32, C12M 1/12, C12M 1/42, C12M 1/36

(54) **VERSUCHSANORDNUNG ZUM UNTERSUCHEN EINER ZELLKULTUR UNTER DYNAMISCHER KRAFTEINWIRKUNG**
TESTING ARRANGEMENT FOR EXAMINING A CELL CULTURE UNDER THE EFFECT OF A DYNAMIC FORCE
AGENCEMENT EXPÉRIMENTAL POUR L'ANALYSE D'UNE CULTURE DE CELLULES SOUS L'APPLICATION D'UNE FORCE DYNAMIQUE

(30) Priorität: 09.06.2015 DE 102015109087
(43) Veröffentlichungstag der Anmeldung: 18.04.2018
(73) Patentinhaber: Technische Universität Darmstadt, 64289 Darmstadt (DE)
(72) Erfinder: MECKEL, Tobias, 64297 Darmstadt (DE); SAPPER, Eva, 69509 Mörlenbach (DE); SCHMITT, Ljubomira Ana, 64319 Pfungstadt (DE)
(74) Vertreter: Habermann Intellectual Property Partnerschaft von Patentanwälten mbB
(86) Internationale Anmeldenummer: PCT/EP2016/063082
(87) Internationale Veröffentlichungsnummer: WO 2016/198482

(56) Entgegenhaltungen:
- WO-A1-2015/038789
- DE-A1- 10 021 627
- DE-A1- 10 151 822
- US-A- 5 827 729
- US-A1- 2013 059 324
- US-A1- 2014 038 258
- PR GBP E D ET AL: "High-Frequency Vibration Treatment of Human Bone Marrow Stromal Cells Increases Differentiation toward Bone Tissue", BONE MARROW RESEARCH, HINDAWI PUBLISHING CORPORATION, US, Bd. 2013, 31. Januar 2013 (2013-01-31), Seiten 803450-1, XP002747338, ISSN: 2090-2999, DOI: 10.1155/2013/803450

## Beschreibung

Die Erfindung betrifft eine Versuchsanordnung zum Untersuchen einer Zellkultur unter dynamischer Krafteinwirkung mit einer Trägerstruktur zur Aufnahme einer Zellkultur und mit einer Krafteinwirkungsvorrichtung.

Viele grundlegende Konzepte zur Übertragung von Informationen zwischen Zellen untereinander sowie zwischen Zellen und ihrer Umgebung erfolgten an Zellkulturen, die außerhalb eines Lebewesens "in vitro" beispielsweise in einer Petrischale herangezüchtet und kultiviert wurden. Die "in vitro" hergestellten und untersuchten Zellkulturen weisen regelmäßig eine flächige, zweidimensionale Anordnung einer großen Anzahl von Zellen auf, die eine Zellkultur bilden. Einzelne Zellen sowie die gesamte Zellkultur können bei der zweidimensionalen Anordnung der Zellkultur in einfacher Weise beispielsweise mit optischen Mikroskopen oder mit anderen geeigneten Messgeräten untersucht und erforscht werden.

Es hat sich gezeigt, dass sich das Verhalten und die Untersuchungsergebnisse einer im Wesentlichen zweidimensionalen Zellkultur nicht ohne weiteres auf lebende Organismen übertragen lassen, bei denen dreidimensional miteinander verbundene Zellen ein natürliches biologisches System bzw. Gewebestrukturen bilden. Es ist bekannt, dass durch eine Anordnung von körpereigenen Zellen auf geeigneten Trägerstrukturen und deren gezielte Vermehrung und Verknüpfung "in vitro" stabile Gewebestrukturen erzeugt werden können, die beispielsweise als künstliches Bindegewebe oder Epithelgewebe ein für die Implantation geeignetes Ersatzgewebe bilden.

Weiterführende Untersuchungen haben ergeben, dass einzelne Aspekte wie beispielsweise das Zellwachstum, die Zellproliferation, die Zellmigration und die Zelldifferenzierung jeweils komplexe Prozesse darstellen, die von intrazellulären Faktoren sowie von extrazellulären Faktoren abhängen. So können beispielsweise neben molekularen bzw. biochemischen Signalen und Umgebungsbedingungen auch mechanische Kräfte sowie elektrische Signale einen Einfluss auf die Entwicklung der Zellen nehmen.

Es hat sich weiterhin gezeigt, dass sich das Zellverhalten einzelner Zellen in einer zweidimensionalen Anordnung deutlich von dem Zellverhalten vergleichbarer Zellen in einer dreidimensionalen Zellkultur unterscheidet, die in einer geeigneten Trägerstruktur erzeugt wird und heranwächst. Beispielsweise wirkt sich die mechanische Steifigkeit der Trägerstruktur unter anderem auf die Mechanosensorik, die Zelladhäsion und die Zellmigration aus und beeinflusst auch die Diffusion von gelösten Substanzen und die Bindung von Proteinen, so dass in Abhängigkeit der Trägerstruktur bestimmte Zellen in ihrer Morphogenese und Proliferation beeinflusst werden.

Um den Einfluss der verschiedenen üblicherweise verwendeten Trägerstrukturen und der Umgebungsbedingungen auf Zellkulturen besser untersuchen zu können kann die Zellkultur während der Kultivierung einer dynamischen Krafteinwirkung ausgesetzt werden. In US 2014 038 258 A ist beispielsweise ein Bioreaktor beschrieben, bei dem auf einer in einer Nährlösung angeordnete Zellkultur eine elektrische Stimulation sowie eine mechanische Kraftweinwirkung ausgeübt werden kann. Die mechanische Krafteinwirkung wird durch einen verlagerbaren Kolben erzeugt, der auf eine auslenkbare dünne Membran einwirkt. Auf der Oberfläche der dünnen Membran wachsen Zellen, die durch die Auslenkung der Membran gedehnt werden können.

In US 2013 059 324 A wird ein ähnliches Wirkprinzip in einer Mikrotiterplatte zur Untersuchung einer großen Anzahl von Zellkulturen beschrieben, bei der in den einzelnen Kavitäten der Mikrotiterplatte Zellen auf der Oberfläche einer auslenkbaren, dehnbaren Membran kultiviert werden. Mit einer an die Mikrotiterplatte angepassten Stempelplatte kann in allen Kavitäten dieselbe Dehnung der Membran und damit der jeweiligen zweidimensionalen Zellkultur erzeugt werden.

In DE 101 51 822 A1 wird eine Vorrichtung zur elektrischen und mechanischen Stimulierung von Zellen beschrieben, die auf eine Oberfläche eines Trägers aufgebracht sind und der Träger mit bewegbaren Klammern in einer Kulturkammer gehalten und durch eine mit einem Motor erzeugte Auslenkung der Klammern einer mechanischen Belastung ausgesetzt wird. Durch die Ausgestaltung der Vorrichtung und insbesondere der bewegbaren Klammern kann eine optische Untersuchung und Beobachtung der Zellen nur aus einer großen Entfernung und deshalb mit einer auf das 10- bis 20-fache beschränkten Vergrößerung der optischen Abbildung durchgeführt werden.

In WO2015/038789 wird ein Verfahren und eine Vorrichtung zur Kultivierung von Zellgewebe unter dynamischer Krafteinwirkung in Form von Vibrationen offenbart. Die Vorrichtung weist einen translationsfähigen Träger in einem festen Haltegestell auf, der das Zellkulturgewebe beherbergt.

Die aus der Praxis bekannten Vorrichtungen ermöglichen oftmals nur eine Krafteinwirkung auf eine flächige bzw. zweidimensionale Zellenanordnung, die beispielsweise auf einer auslenkbaren dehnbaren Membran oder auf einem mechanisch beanspruchbaren Träger aufgebracht ist. Dabei kann in den meisten Fällen ausschließlich in eine Richtung eine Kraft auf die flächige Zellanordnung ausgeübt werden, so dass beispielsweise durch die Elastizität der ausgelenkten Membran eine Rückstellung in eine Ausgangslage erfolgt. Um die gewünschte Krafteinwirkung erzeugen zu können sind regelmäßig konstruktiv aufwendige Apparaturen erforderlich, die eine gleichzeitige Untersuchung der flächigen Zellanordnung beeinträchtigen oder unmöglich werden lassen. Deshalb muss eine Zellkultur zur Untersuchung dieser Zellkultur aus einer derartigen Vorrichtung entnommen und einer geeigneten Untersuchungsapparatur zugeführt werden.

Es wird als eine Aufgabe der vorliegenden Erfindung angesehen, eine Versuchsanordnung zum Untersuchen einer Zellkultur unter dynamischer Krafteinwirkung so auszugestalten, dass nicht nur zweidimensional auf einer Oberfläche einer Trägerstruktur angeordnete Zellkulturen einer mechanischen Krafteinwirkung ausgesetzt werden können, dass die Krafteinwirkung möglichst präzise gesteuert und kontrolliert werden kann und dass auch eine Untersuchung der Zellkultur während einer Krafteinwirkung durchgeführt werden kann.

Diese Aufgabe wird erfindungsgemäß gelöst durch eine Versuchsanordnung gemäß Ansprüchen 1 und 18 mit einer Trägerstruktur, die dreidimensional ausgebildet und so ausgestaltet ist, dass die Zellkultur in die Trägerstruktur eingebettet ist, wobei durch eine Krafteinwirkung eine Verformung der Trägerstruktur bewirkt werden kann und dass die Krafteinwirkungsvorrichtung einen ersten Aktor aufweist, der beabstandet von der in der Trägerstruktur eingebetteten Zellkultur angeordnet und so ausgerichtet ist, dass der erste Aktor eine gerichtete Kraftwirkung auf die Trägerstruktur ausüben kann.

Die Trägerstruktur kann beispielsweise aus einem geeigneten Elastomer hergestellt und mittels Polymerisation oder mit Hilfe von dreidimensionalen Druckverfahren als dreidimensionale Gerüststruktur ausgebildet werden. Die Zellkultur ist derart in die Trägerstruktur eingebettet, dass durch eine erzwungene Verformung der Trägerstruktur eine Krafteinwirkung auf die in der Trägerstruktur eingebettete Zellkultur ausgeübt wird.

Als Trägerstruktur kann vorteilhafterweise ein verformbares elastisches bzw. faserhaltiges Gebilde aus einem geeigneten Polymer wie beispielsweise Silikon oder Latex, aber auch aus Kollagen oder Gelatine bestehen oder diese Materialien in Kombination mit weiteren Trägermaterialien aufweisen. Es ist ebenfalls möglich, dass ein natürliches Gewebe wie beispielsweise ein Herzmuskelgewebe oder ein künstliches Biogewebematerial als Trägerstruktur verwendet wird. Nachfolgend werden noch weitere geeignete Trägerstrukturen beschrieben. Die Trägerstruktur kann durch geeignete Verfahren wie beispielsweise eine Copolymerisation, eine Plasmabehandlung, ein Ätzvorgang oder eine Bestrahlung sowie mit Hilfe von chemischen oder biologischen Substanzen behandelt werden, um die Anhaftung von Zellen der Zellkultur an der Trägerstruktur zu begünstigen.

Die Krafteinwirkungsvorrichtung mit einem ersten Aktor wirkt beabstandet von der in der Trägerstruktur eingebetteten Zellkultur auf die Trägerstruktur ein. Die Krafteinwirkung erfolgt demzufolge in einer Art und Weise, die den Verhältnissen in nativen Zellgeweben und Zellumgebungen nachgebildet ist und deshalb realistische und aussagekräftige Untersuchungsergebnisse ermöglicht.

Der beabstandet von der Zellkultur angeordnet Aktor ermöglicht eine mechanische Belastung der Zellkultur und erlaubt gleichzeitig eine Untersuchung der mechanisch belasteten Zellkultur beispielsweise mit hochauflösenden optischen Mikroskopen oder Fluoreszenzmikroskopen. Durch eine geeignete Ausgestaltung der Trägerstruktur kann erreicht werden, dass sich die Trägerstruktur bei einer Betätigung des Aktors über einen großen räumlichen Bereich hinweg verformt, so dass ein entsprechend großer Abstand des Aktors von der Zellkultur vorgegeben werden kann. Durch einen großen Abstand des Aktors von der Zellkultur kann eine Analyseneinrichtung wie beispielsweise ein optisches Mikroskop sehr nahe an die Trägerstruktur und damit einhergehend sehr nahe an die zu untersuchende Zellkultur angenähert werden, so dass beispielsweise die Verwendung eines Mikroskop-Objektivs mit einer 100-fachen Vergrößerung mit einer numerischen Apertur von mehr als 1.4 möglich ist.

Gemäß der vorliegenden Erfindung ist vorgesehen, dass die Krafteinwirkungsvorrichtung einen zweiten Aktor aufweist, der beabstandet von der in der Trägerstruktur eingebetteten Zellkultur auf die Trägerstruktur einwirkt, und dass der erste Aktor und der zweite Aktor eine voneinander abweichend gerichtete Krafteinwirkung auf die Trägerstruktur ausüben. Der erste Aktor und der zweite Aktor können beispielsweise senkrecht zueinander ausgerichtet sein bzw. eine senkrecht zueinander gerichtete Krafteinwirkung erzeugen. In diesem Fall können mit dem ersten Aktor und dem zweiten Aktor durch eine geeignete Überlagerung der jeweiligen Krafteinwirkung beliebige Kraftwirkungsrichtungen in einer durch den ersten Aktor und den zweiten Aktor vorgegebenen Kraftwirkungsebene erzeugt und auf die Zellkultur ausgeübt werden.

Der erste Aktor und der zweite Aktor können unabhängig voneinander sowie in unterschiedlicher Art und Weise betätigt werden. Beispielsweise kann mit dem ersten Aktor eine zyklische Kompression mit einer vorgebbaren Frequenz auf die Trägerstruktur und damit auf die Zellkultur ausgeübt werden, während mit dem zweiten Aktor eine gleichbleibende oder in deutlich größeren Zeitabständen wechselnde Druckbelastung auf die Trägerstruktur bzw. auf die Zellkultur ausgeübt wird. Die Richtungen der Krafteinwirkung des ersten Aktors und des zweiten Aktors können auch einen beliebigen Winkel zwischen 0° und 180° relativ zueinander aufweisen.

Es ist vorgesehen, dass die Krafteinwirkungsvorrichtung einen dritten Aktor aufweist, der ebenfalls beabstandet von der in der Trägerstruktur eingebetteten Zellkultur auf die Trägerstruktur einwirkt, und dass der dritte Aktor eine von dem ersten Aktor und von dem zweiten Aktor abweichend gerichtete Krafteinwirkung auf die Trägerstruktur ausübt. Durch eine geeignete Anordnung und Ausrichtung der drei Aktoren können nahezu beliebig gerichtete dreidimensional auf die Trägerstruktur einwirkende Kräfte erzeugt werden. Zudem kann die resultierende Gesamtkraft, die auf die Zellkultur einwirkt, mit Hilfe der drei Aktoren in allen Raumrichtungen präzise vorgegeben und zuverlässig ausgeübt werden. Durch die Verwendung von drei Aktoren, die in unterschiedlichen Richtungen auf die Trägerstruktur einwirken, können komplexe und lebensnahe mechanische Beanspruchungen der Zellkultur simuliert und mit zeitgleich oder nachfolgend durchgeführten Untersuchungen ausgewertet werden.

Um eine möglichst präzise vorgebbare Krafteinwirkung bei möglichst gut kontrollierbaren und reproduzierbaren Umgebungsbedingungen ausüben zu können ist vorgesehen, dass die Versuchsanordnung ein Haltegestell zur Aufnahme der Trägerstruktur aufweist und dass mindestens einer der Aktoren derart an dem Haltegestell festgelegt ist, dass der Aktor eine die Trägerstruktur verformende Krafteinwirkung auf die in dem Haltegestell aufgenommene Trägerstruktur ausüben kann.

Gemäß einer vorteilhaften Ausgestaltung des Erfindungsgedankens ist vorgesehen, dass der an dem Haltegestell festgelegte Aktor mit einer ersten Seite der Trägerstruktur in Wirkverbindung steht, und dass die Trägerstruktur an einer der ersten Seite gegenüberliegenden zweiten Seite mit einer Befestigungseinrichtung an dem Haltegestellt festgelegt ist, so dass durch den Aktor Zugkräfte oder Druckkräfte auf die Trägerstruktur übertragen werden können. Die Trägerstruktur kann beispielsweise klemmend in der Befestigungseinrichtung an dem Haltegestellt festgelegt sein. Es ist ebenfalls möglich, die Trägerstruktur mit einem geeigneten Klebemittel oder Haftmittel an einer Haftfläche der Befestigungseinrichtung zu befestigen. Auch eine formschlüssige Festlegung der Trägerstruktur an der Befestigungseinrichtung ist denkbar und für bestimmte Anwendungsfälle bzw. Trägerstrukturen vorteilhaft.

Mit einem linear verstellbaren Aktor können in einfacher Weise präzise vorgebbare Zugkräfte oder Druckkräfte auf die Trägerstruktur übertragen werden. Längs einer vorgegebenen Richtung im Wesentlichen eindimensional verstellbare Aktoren, die einen ausreichend großen Verstellweg sowie einen ausreichend kraftvollen Stellantrieb aufweisen, sind in zahlreichen Varianten handelsüblich und kostengünstig erhältlich.

Es ist ebenfalls möglich, dass die Befestigungseinrichtung an der zweiten Seite der Trägerstruktur einen weiteren Gegenaktor aufweist, der an dem Haltegestell festgelegt ist und ebenfalls eine Kraftwirkung auf die Trägerstruktur ausüben kann. Der weitere Gegenaktor kann ebenso wie der erste Aktor, der an der ersten Seite der Trägerstruktur angeordnet ist und auf die Trägerstruktur einwirkt, eine im Wesentlichen lineare Krafteinwirkung auf die Trägerstruktur ausüben, die entweder in entgegengesetzter Richtung oder gleichgerichtet zu der Krafteinwirkung des ersten Aktors ist. Bei einer gleichzeitigen Krafteinwirkung in entgegengesetzter Richtung wird eine verstärkte Kompression oder Dehnung der Trägerstruktur herbeigeführt.

Bei einer gleichgerichteten Krafteinwirkung wird die Trägerstruktur von den beiden Aktoren, dem ersten Aktor und dem zugeordneten Gegenaktor, verlagert und dabei beschleunigt, so dass die in der Trägerstruktur eingebettete Zellkultur vor allem Beschleunigungskräften ausgesetzt ist, die durch die Verlagerung der Trägerstruktur erzeugt werden. Ebenso wie der erste Aktor können auch der zweite Aktor und der dritte Aktor jeweils mit an jeweils gegenüberliegenden Seiten angeordneten Befestigungseinrichtungen kombiniert werden. Die Befestigungseinrichtungen können ebenfalls jeweils einen weiteren Gegenaktor aufweisen. Um eine möglichst umfassende Kontrolle und möglichst präzise Vorgabe der mechanischen Beanspruchung der Zellkultur zu ermöglichen, können in allen drei Raumrichtungen an jeweils zwei gegenüberliegenden Seiten der Trägerstruktur jeweils ein Aktor und ein Gegenaktor so angeordnet sein, dass mit diesen Aktoren beabstandet zur Zellkultur eine die Trägerstruktur verformende Krafteinwirkung erzeugen kann. Auch die Verwendung von weniger als sechs Aktoren oder von mehr als sechs Aktoren kann für Untersuchungen der Zellkultur mit möglichst realistisch simulierten dreidimensionalen Krafteinwirkungen zweckmäßig sein.

Um gleichzeitig eine mechanische Beanspruchung und eine optische Untersuchung der Zellkultur durchführen zu können ist vorgesehen, dass das Haltegestell als Rahmengestell ausgebildet ist und mindestens an zwei gegenüberliegenden Gestellseiten optisch durchsichtige Öffnungen aufweist, durch welche die Trägerstruktur beleuchtet und betrachtet werden kann. Das Haltegestell kann beispielsweise als Gitterkäfig ausgebildet sein und von allen Seiten eine nahezu ungehinderte Betrachtung der Trägerstruktur und der darin eingebetteten Zellkultur ermöglichen. Es ist ebenso möglich, das Haltegestell aus mehreren miteinander verbundenen Stäben zusammengesetzt ist, die ein Fachwerk bilden. Im Hinblick auf eine eventuell gewünschte Abschirmung der Trägerstruktur kann es vorteilhaft sein, dass das Haltegestell ein nahezu allseitig geschlossenes Gehäuse aufweist, das eine Mess- und Untersuchungskammer umgibt, in welcher die Trägerstruktur mechanischen Beanspruchungen ausgesetzt werden kann und Untersuchungen an der Trägerstruktur durchgeführt werden können.

Das Haltegestell kann auch eine flüssigkeitsdichte Probenkammer zur Aufnahme der Trägerstruktur mit der Zellkultur aufweisen. Die Aktoren können in einem Innenraum der Probenkammer angeordnet und an Kammerwänden der Probenkammer oder dafür vorgesehenen Haltestrukturen festgelegt sein. Es ist ebenfalls denkbar, dass die Aktoren außerhalb der Probenkammer angeordnet sind und durch abgedichtete Öffnungen mit der Trägerstruktur in Wirkverbindung stehen, die sich in der Probenkammer befindet. Um eine hochauflösende optische Abbildung und Untersuchung der Zellkultur zu erleichtern kann ein Mikroskop-Objektiv an oder in der Probenkammer angeordnet sein, das mit einer optischen Abbildungseinrichtung oder Analyseneinrichtung verbunden ist oder verbunden werden kann.

In vorteilhafter Weise ist vorgesehen, dass der erste Aktor und gegebenenfalls weitere Aktoren und/oder Gegenaktoren verlagerbar an dem Rahmengestell gelagert sind. Die verlagerbar an dem Rahmengestell gelagerten Aktoren und gegebenenfalls Gegenaktoren können hinsichtlich ihrer jeweiligen Positionierung an Trägerstrukturen mit unterschiedlichen Abmessungen angepasst werden. Zudem kann eine von den Aktoren und gegebenenfalls Gegenaktoren kraftschlüssig oder formschlüssig ergriffene Trägerstruktur durch eine Verlagerung der Aktoren und gegebenenfalls Gegenaktoren ihrerseits verlagert werden, um beispielsweise automatisiert von einer Zellkultivierungsposition in einer Nährlösung in eine Analysenposition an oder in einer Analysenvorrichtung bewegt zu werden.

Gemäß einer besonders vorteilhaften Ausgestaltung des Erfindungsgedankens ist vorgesehen, dass die Trägerstruktur mindestens eine Ausnehmung aufweist, in welche der erste Aktor eingreifen kann, um durch eine Krafteinwirkung die Verformung der Trägerstruktur zu bewirken. Durch die Ausbildung einer Ausnehmung in der Trägerstruktur kann der Aktor beispielsweise einen stabförmigen oder lanzenförmigen Aktorabschnitt aufweisen, der durch eine lineare Verlagerung des stabförmigen oder lanzenförmigen Aktorabschnitts in die Ausnehmung eingebracht und gegebenenfalls auch wieder aus der Ausnehmung entfernt werden kann. Durch die Ausnehmung kann in einfacher Weise eine formschlüssige Verbindung der Trägerstruktur mit dem Aktor erreicht und während der Dauer der Krafteinwirkung beibehalten werden.

Zweckmäßigerweise ist vorgesehen, dass die Trägerstruktur mindestens zwei Ausnehmungen aufweist, die in einem Winkel zueinander beabstandet zueinander so angeordnet sind, dass ein erster Aktor und ein zweiter Aktor auf gegenüberliegenden Seiten der Zellkultur in jeweils eine zugeordnete Ausnehmung eingreifen können, um die Trägerstruktur ergreifen und von einem Untergrund abheben zu können. Durch eine nicht parallele, sondern in einem Winkel zueinander ausgerichtete Anordnung der Ausnehmungen können der erste Aktor und der zweite Aktor mit stabförmigen Aktorabschnitten oder mit an die Ausgestaltung der Ausnehmungen angepassten Eingriffselementen derart mit der Trägerstruktur in Eingriff gebracht werden, dass die Trägerstruktur mit nur zwei Aktoren auch ergriffen und beispielsweise von einem Untergrund angehoben werden kann, um eine Vielzahl unterschiedlicher Krafteinwirkungen zu realisieren. Zudem kann die Trägerstruktur mit der Krafteinwirkungseinrichtung auch ergriffen und verlagert werden, um beispielsweise die Trägerstruktur automatisiert aus einer Vorrats- oder Lagereinrichtung in eine Kultivierungseinrichtung oder in eine Analyseneinerichtung, bzw. in die Versuchsanordnung zu überführen.

Um die Ausnehmung besonders einfach und kostengünstig herstellen zu können ist vorgesehen, dass die mindestens eine Ausnehmung in der Trägerstruktur taschenförmig ausgestaltet ist.

Mit der erfindungsgemäßen Versuchsanordnung kann mit der Krafteinwirkungsvorrichtung während der Kultivierung von Zellen und während der Untersuchung bzw. Analyse der Zellen eine in beliebigen Richtungen auf die Trägerstruktur und dadurch auf die darin eingebetteten Zellen einwirkende Kraft erzeugt werden. Im Hinblick auf eine effiziente Analyse einer großen Anzahl von Zellen und Zellkulturen ist einer vorteilhaften Ausgestaltung des Erfindungsgedankens zufolge vorgesehen, dass das Haltegestell mehrere Krafteinwirkungseinrichtungen mit jeweils mindestens einem ersten Aktor und mehrere Trägerstrukturen aufweist, wobei mit den mehreren ersten Aktoren jeweils eine die mindestens eine zugeordnete Trägerstruktur verformende Krafteinwirkung auf die betreffende Trägerstruktur ausgeübt wird. Durch die Anordnung von einem ersten Aktor und einem zweiten Aktor sowie gegebenenfalls einem dritten Aktor und jeweils diesen Aktoren gegenüberliegend angeordneten weiteren Gegenaktoren, die jeweils einer Trägerstruktur zugeordnet sind, können auf jede Trägerstruktur gleichzeitig und gegebenenfalls unabhängig voneinander aus allen Richtungen einwirkende Kräfte ausgeübt und dadurch komplexe Verformungen der betreffenden Trägerstrukturen erzwungen werden. Auf diese Weise kann eine große Anzahl von Trägerstrukturen gleichzeitig kultiviert und analysiert werden und während dieser Behandlungsdauer beliebigen Kräften ausgesetzt werden.

Um die Verwendung von Mikrotiterplatten zu ermöglichen ist vorgesehen, dass das Haltegestell eine Verbindungseinrichtung zur Verbindung mit einer Mikrotiterplatte aufweist, so dass das Haltegestell derart mit der Mikrotiterplatte verbunden werden kann, dass jede Trägerstruktur in einer zugeordneten Kavität der Mikrotiterplatte angeordnet und mit dem zugeordneten der betreffenden Trägerstruktur zugeordneten ersten Aktor verformt werden kann. Die Verbindungseinrichtung kann auch eine Aufnahmeeinrichtung, beispielsweise ein Fach oder eine Schublade, zur Aufnahme einer Mikrotiterplatte aufweisen. Einer Ausgestaltung des Erfindungsgedankens zufolge ist vorgesehen, dass mindestens einer der Aktoren ein Piezoaktor ist. Aus der Praxis sind Piezoaktoren mit einer Piezokeramik bekannt, mit denen Auslenkungen von bis zu 2 mm bei kurzen Ansprechzeiten von etwa 20 Millisekunden und einer niedrigen Betriebsspannung von weniger als 50 Volt realisiert werden können. Derartige Piezoaktoren können dauerhaft präzise angesteuert und ausgelenkt werden, um über einen großen Zeitraum eine genauere produzierbare Krafteinwirkung auf die Trägerstruktur ausüben zu können.

Es ist ebenfalls denkbar, dass mindestens einer der Aktoren einen Formgedächtniswerkstoff aufweist. Mit Hilfe von Formgedächtnislegierungen oder Formgedächtnispolymeren können mechanische Auslenkungen eines Stellglieds beispielsweise elektrisch oder thermisch stimuliert werden. Es ist ebenfalls denkbar, dass mindestens einer der Aktoren ein elektrothermisch betätigbarer Polymeraktor ist.

Um die mit den Aktoren auf niedriger Struktur ausgeübten Kräfte und mechanischen Beanspruchungen nachweisen zu können ist vorgesehen, dass in den Aktoren und/oder in der Trägerstruktur mindestens eine Sensoreinrichtung zur Erfassung der auf die Trägerstruktur einwirkenden Kräfte angeordnet ist. Die Sensoreinrichtung kann beispielsweise einen Positionssensor oder einen Wegsensor aufweisen und eine relative oder absolute Verlagerung bzw. Verformung der Trägerstruktur erfassen, so dass über Referenzmessungen und Vergleichswerte die auf die Trägerstruktur einwirkende Kraft ermittelt werden kann. Es ist ebenfalls möglich, miniaturisierte Druck- oder Kraftmesssensoren an oder in der Trägerstruktur anzuordnen.

Aus der Praxis sind auch Elastomere bekannt, bei denen eine Kompression oder Dehnung des Elastomers optisch detektiert werden kann.

Es ist ebenfalls möglich, mindestens einen Aktor in einem Abstand von der Zellkultur auf der Trägerstruktur so anzuordnen und festzulegen, dass durch eine Betätigung des Aktors eine Verformung der Trägerstruktur bewirkt wird. Es sind elektroaktive Polymere bekannt, bei denen durch die Anlegung einer sich verändernden Spannung oder sich verändernden Ladung die Gestalt der elektroaktiven Polymere verändert werden kann. Dazu zählen sowohl ionische elektroaktive Polymere wie beispielsweise leitfähige Polymere, ionische Metall-Polymer-Komposite oder ionische Gele als auch elektronische elektroaktive Polymere wie beispielsweise elektrostriktive und ferroelektrische Polymere sowie dielektrische Elastomeraktoren. Mit derartigen elektroaktiven Polymeren können beispielsweise flächige Aktorstreifen erzeugt werden, die in einem Abstand zu der Zellkultur auf der Trägerstruktur angeordnet und flächig verbunden werden können. Durch die Anlegung einer geeigneten Wechselspannung wird eine alternierende Expansion und Kontraktion des Aktorstreifens bewirkt, die sich unmittelbar auf die damit verbundene Trägerstruktur überträgt und eine entsprechende Dehnung bzw. Kontraktion der Trägerstruktur erzwingt. Der Aktorstreifen ist dabei ausschließlich mit der Trägerstruktur verbunden, ohne dass ein Haltegestell zur Festlegung des Aktorstreifens erforderlich ist. Das elektroaktive Polymer kann auch eine von einem Streifen abweichende Formgebung aufweisen, die an die gewünschte Verformung der Trägerstruktur angepasst ist. In vorteilhafter Weise ist vorgesehen, dass mehrere Aktoren rahmenförmig die Zellkultur umgebend auf einer Oberfläche der Trägerstruktur angeordnet sind. Dadurch können bei einer geeigneten Betätigung der Aktoren in einem die Zellkultur umgebenden Bereich der Trägerstruktur Längs- und Querkontraktionen in die Trägerstruktur eingebracht werden, die durch die Trägerstruktur auf die Zellkultur übertragen werden. Durch die rahmenförmige Anordnung kann eine gleichmäßige und kontrollierbare Verformung auf den von der rahmenförmigen Anordnung umgebenen Bereich der Trägerstruktur ausgeübt werden.

Die Aktoren können beispielsweise mit geeigneten Druckverfahren auf die Trägerstruktur aufgedruckt werden. Es ist ebenfalls möglich, die Aktoren mit der Trägerstruktur kraft- oder formschlüssig zu verbinden, bzw. zu verkleben. Bei einer Trägerstruktur mit einer dreidimensional strukturierten Oberfläche kann auch eine formschlüssige Verbindung der Aktoren mit der Trägerstruktur verwirklicht werden. Die Trägerstruktur kann auch eine beispielsweise streifenförmige Ausnehmung aufweisen, in welche der zugeordnete Aktor eingebettet ist.

Neben elektroaktiven Polymeren sind zahlreiche weitere Aktoren bzw. Wirkmechanismen bekannt, die ebenfalls dazu geeignet sind, entweder in Kombination mit einem Haltegestell oder ausschließlich mit der Trägerstruktur verbunden zu sein und bei einer Betätigung eine Verformung der Trägerstruktur zu bewirken. So können beispielsweise elektromechanische, elektrochemische, magnetostriktive, hydraulische oder pneumatische, Bi-Metall- oder auch elektromagnetische Aktoren, wie z.B. Voice-Coil Aktoren, verwendet werden.

In vorteilhafter Weise ist vorgesehen, dass die Trägerstruktur Fasern aufweist. Die Fasern können Zugkräfte in Faserrichtung über große Bereiche und bei einer geeigneten Verbundwirkung der Fasern untereinander weit über eine Faserlänge hinweg übertragen. Eine Fasern aufweisende Trägerstruktur kann in einfacher Weise durch von außen einwirkende Kräfte weiträumig verformt werden. Zwischen einzelnen Fasern kann die Zellkultur in der Trägerstruktur eingebettet sein.

Der für die Trägerstruktur verwendete Werkstoff kann porös ausgestaltet sein und eine einfache Zuführung von Nährlösungen sowie gelöster chemischer oder biologischer Substanzen zu der Zellkultur erleichtern. Der Werkstoff kann für optische Untersuchungen ausreichend durchsichtig sein, so dass eine mikroskopische Untersuchung der in der Trägerstruktur eingebetteten Zellkultur nahezu ungehindert möglich ist. Eine große Anzahl verschiedener Werkstoffe mit unterschiedlichen Eigenschaften ist handelsüblich und kostengünstig erhältlich.

Gemäß einer besonders vorteilhaften Ausgestaltung des Erfindungsgedankens ist vorgesehen, dass in die Trägerstruktur ein biokompatibles Hydrogel zur Aufnahme der Zellkultur eingebettet ist.

Das Hydrogel kann in der Trägerstruktur eine Matrix bilden, in welche die Zellkultur eingebettet ist. Ein geeignetes Hydrogel kann beispielsweise ein Kollagen, Gelatine oder ein Polyethylenglykol sein. Das Hydrogel kann auch Laminin, Fibronektin oder Hyaluronsäure aufweisen oder im Wesentlichen daraus bestehen. Das Hydrogel kann die Krafteinwirkung der mit den Aktoren verformten Trägerstruktur auf die Zellkultur übertragen. Durch das Hydrogel können für das Heranwachsen der Zellkultur vorteilhafte Umgebungsbedingungen vorgegeben werden.

Nachfolgend werden Ausführungsbeispiele des Erfindungsgedankens näher erläutert, die in der Zeichnung dargestellt sind. Es zeigt:
Fig. 1 eine schematische perspektivische Darstellung der Versuchsanordnung mit einer quaderförmigen Trägerstruktur, die mit Aktoren in einem die Trägerstruktur umgebenden Haltegestell festgelegt ist,
Fig. 2 eine schematische Schnittansicht der in Fig. 1 gezeigten Versuchsanordnung längs der horizontalen Ebene II-II in Fig. 1,
Fig. 3 eine schematische Schnittansicht einer abweichend ausgestalteten Versuchsanordnung, bei der ein Haltegestell auf einer Mikrotiterplatte angeordnet ist, in der mehrere Trägerstrukturen angeordnet und durch mehrere Krafteinwirkungseinrichtungen verformt werden können,
Fig. 4 eine Draufsicht auf die in Fig. 3 gezeigte Versuchsanordnung,
Fig. 5 eine schematische Schnittansicht einer Trägerstruktur, die von einer Krafteinwirkungseinrichtung mit einem ersten Aktor, einem zweiten Aktor und einem dritten Aktor gehalten wird,
Fig. 6 eine schematische Seitenansicht der in Fig. 5 dargestellten Trägerstruktur mit der zugeordneten Krafteinwirkungseinrichtung,
Fig. 7 eine schematische Ansicht einer flachen quaderförmigen Trägerstruktur, auf der vier Aktoren rahmenförmig angeordnet sind,
Fig. 8 eine Draufsicht auf die in Fig. 7 gezeigte Versuchsanordnung,
Fig. 9 eine schematische Schnittansicht längs der Linie IX-IX in Fig. 8, und
Fig. 10 eine schematische Schnittansicht gemäß Fig. 9, wobei der Aktor abweichend zu dem in Fig. 9 gezeigten Ausführungsbeispiel auf der Trägerstruktur festgelegt ist.

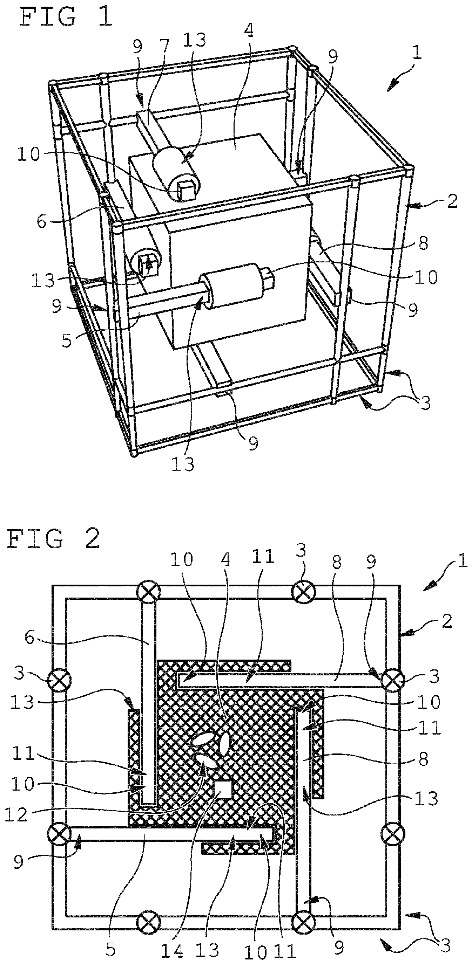

Eine in den Figuren 1 und 2 schematisch dargestellte Versuchsanordnung 1 weist ein Haltegestell 2 auf, das aus mehreren rechtwinklig miteinander verbundenen und sich kreuzenden Stäben 3 zusammengesetzt ist. In dem Haltegestell 2 ist eine Trägerstruktur 4 angeordnet, die über einen ersten Aktor 5, einen zweiten Aktor 6 und einen dritten Aktor 7 sowie über drei weitere Gegenaktoren 8 in dem Haltegestell 2 gelagert ist. Der erste Aktor 5, der zweite Aktor 6 und der dritte Aktor 7 sind jeweils rechtwinklig zueinander angeordnet, so dass über diese drei Aktoren 5, 6 und 7 Kräfte aus bzw. in einer beliebigen Raumrichtung auf die Trägerstruktur 4 ausgeübt werden können. Jedem Aktor 5, 6 und 7 ist ein Gegenaktor 8 zugeordnet, der auf einer dem betreffenden Aktor 5, 6 und 7 gegenüberliegenden Seite der Trägerstruktur 4 mit der Trägerstruktur 4 in Eingriff steht und entweder eine gleichgerichtete oder entgegengesetzt gerichtete Krafteinwirkung auf die Trägerstruktur 4 ausüben kann.

Die Aktoren 5, 6 und 7 sowie die Gegenaktoren 8 stützen sich jeweils an einem jeweiligen ersten Ende 9 der Aktoren 5, 6 und 7 sowie der Gegenaktoren 8 an einem jeweils zugeordneten Stab 3 des Haltegestells 2 ab. Die Aktoren 5, 6 und 7 sowie die Gegenaktoren 8 sind balkenförmig ausgebildet und weisen jeweils einen beabstandet von dem ersten Ende 9 beginnenden und sich bis zu einem dem ersten Ende 9 gegenüberliegenden zweiten Ende 10 erstreckenden Verstellabschnitt 11 auf, der durch eine Betätigung des betreffenden Aktors 5, 6 oder 7 bzw. des Gegenaktors 8 in einer vorgegebenen Weise ausgelenkt oder verformt werden kann. Durch eine Betätigung der Aktoren 5 und 6 wird eine Kraftwirkung auf die Trägerstruktur 4 ausgeübt, die jeweils im Bereich des Verstellabschnitts 11 mit den einzelnen Aktoren 5 und 6 kraftübertragend verbunden ist.

Jeder Aktor 5, 6 und 7 bildet mit seinem zugeordneten Gegenaktor 8 ein Aktorenpaar, mit dem vor allem in einer durch die Anordnung des Aktorenpaars vorgegebenen Raumrichtung eine Krafteinwirkung auf die Trägerstruktur 4 ausgeübt werden kann. Die drei Aktorenpaare sind jeweils senkrecht zueinander ausgerichtet und so angeordnet, dass mit den drei Aktorenpaaren Zug- und Druckkräfte in allen drei Raumrichtungen auf die Trägerstruktur 4 ausgeübt werden können. Das durch die miteinander verbundenen Stäbe 3 gebildete Haltegestell 2 weist eine hierfür ausreichende mechanische Festigkeit auf. Gleichzeitig verdeckt das Haltegestell 2 die darin angeordnete Trägerstruktur 4 nur unmerklich, so dass eine in der Trägerstruktur 4 eingebettete Zellkultur 12 von allen Seiten nahezu ungehindert betrachtet, analysiert und untersucht werden kann.

Durch die Aktoren 5, 6 und 7 sowie durch die Gegenaktoren 8 wird die Trägerstruktur 4 in dem Haltegestell 2 gelagert, wobei durch eine Betätigung der Aktoren 5, 6 und 7 und der Gegenaktoren 8 eine gezielte und steuerbare Krafteinwirkung auf die Trägerstruktur 4 ausgeübt werden kann, so dass die Trägerstruktur 4 verformt wird. Die drei Aktoren 5, 6 und 7 sowie die Gegenaktoren 8 können auch verlagerbar an dem Haltegestell 2 gelagert sein, um eine Verlagerung der von den Aktoren 5, 6 und 7 sowie den Gegenaktoren 8 ergriffenen Trägerstruktur 4 zu ermöglichen.

Um einen gegebenenfalls lösbaren formschlüssigen Eingriff der Aktoren 5, 6 und 7 sowie der Gegenaktoren 8 mit der Trägerstruktur 4 zu ermöglichen weist die Trägerstruktur 4 für jeden Aktor 5, 6 und 7 sowie Gegenaktor 8 jeweils eine zugeordnete taschenförmig ausgestaltete Ausnehmung 13 auf. Die stabförmig, bzw. lanzenförmig ausgestalteten zweiten Enden 10 greifen in die taschenförmig ausgestalteten Ausnehmungen 13 ein. Durch eine Verlagerung der Trägerstruktur 4 oder der Aktoren 5, 6 und 7 sowie Gegenaktoren 8 kann der Eingriff einzelner oder aller Aktoren 5, 6 und 7 sowie der Gegenaktoren 8 mit der Trägerstruktur 4 hergestellt oder gelöst werden.

Durch die von den Aktoren 5 und 6 erzwungene Verformung der Trägerstruktur 4 wird eine mechanische Krafteinwirkung auf die in der Trägerstruktur 4 eingebetteten Zellkulturen 12 ausgeübt. Die Trägerstruktur 4 besteht aus einem faserhaltigen Werkstoff, in welches die Zellkulturen 12 eingebettet sind. Es ist ebenfalls denkbar, mit Hilfe von geeigneten Formgebungsverfahren wie beispielsweise dem selektiven Lasersintern, der Schmelzschichtung oder dreidimensionalen Drucktechniken eine dreidimensionale Gerüststruktur aus einem geeigneten Kunststoff, aus einer Keramik oder aus Metall herzustellen. Dabei können wesentliche Eigenschaften wie die mechanische Steifigkeit, die Porengröße bzw. Porosität oder die Oberflächenbeschaffenheiten gezielt vorgegeben und an die Versuchsanforderungen sowie an die zu untersuchenden Zellkulturen angepasst sein.

In der Trägerstruktur 4 ist zusätzlich zu den Zellkulturen 12 auch eine Sensoreinrichtung 14 eingebettet, mit der die von den Aktoren 5 und 6 auf die Trägerstruktur 4 ausgeübte Krafteinwirkung erfasst werden kann.

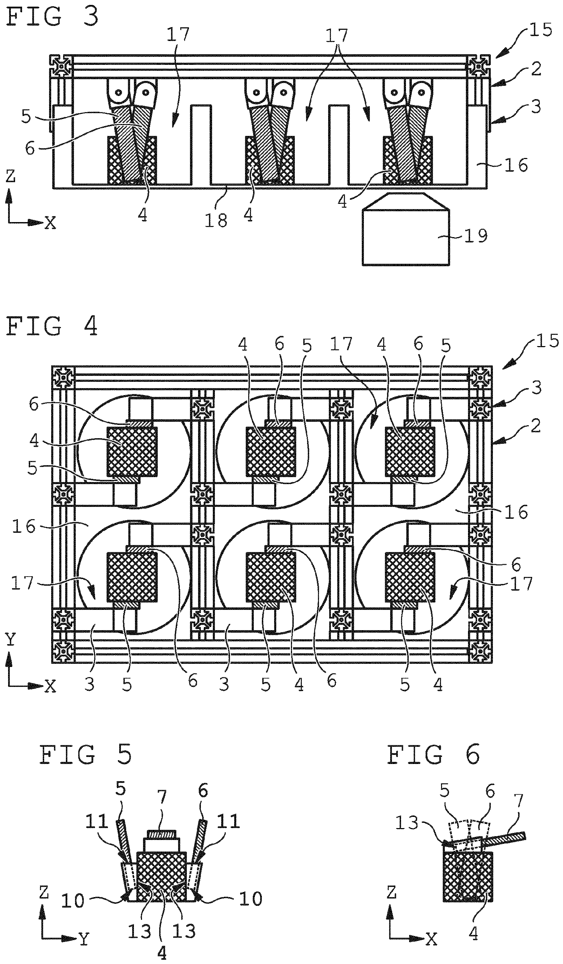

In den Fig. 3 und 4 ist exemplarisch eine abweichend ausgestaltete Versuchsanordnung 15 dargestellt. Das ebenfalls aus Stäben zusammengesetzte Haltegestell 2 ist mit einer Mikrotiterplatte 16 mit sechs voneinander getrennten Kavitäten 17 verbunden. Die Versuchsanordnung weist für jede Kavität 17, in der sich jeweils eine Trägerstruktur 4 befindet, eine zugeordnete Krafteinwirkungseinrichtung mit einem ersten Aktor 5 und einem zweiten Aktor 6 auf, die auf gegenüberliegenden Seiten der Trägerstruktur 4 in einem Winkel zueinander ausgerichtet sind und jeweils in eine taschenförmige Ausnehmung 13 der Trägerstruktur 4 eingreifen. Durch die Anordnung und Ausrichtung des ersten Aktors 5 relativ zu dem zweiten Aktor 6 kann die Trägerstruktur 4 im Raum positioniert, bzw. fixiert werden. Mit dem ersten Aktor 5 und dem zweiten Aktor 6 kann die jeweilige Trägerstruktur 4 demzufolge nicht nur verformt, sondern auch ergriffen und angehoben, bzw. verlagert werden. Der zweite Aktor 6 kann dabei wie ein Gegenaktor 8 der in den Fig. 1 und 2 dargestellten Variante wirken.

Mit der Versuchsanordnung 15 können gleichzeitig sechs verschiedene Zellkulturen 12 in einer jeweils zugeordneten Trägerstruktur 4 kultiviert und analysiert werden, wobei jederzeit eine nahezu beliebige Krafteinwirkung auf die einzelnen Trägerstrukturen 4 mit der jeweils zugeordneten Krafteinwirkungseinrichtung, bzw. mit den zugeordneten Aktoren 5 und 6 ausgeübt werden kann.

Um eine besonders hochauflösende und zuverlässige Untersuchung der Zellkulturen 12 zu ermöglichen ist vorgesehen, dass die Mikrotiterplatte 16 zumindest im Bereich der Kavitäten 17 einen optisch transparenten Bodenbereich 18 aufweist, so dass ein Objektiv 19 einer nicht näher dargestellten optischen Untersuchungseinrichtung sehr nahe an der Trägerstruktur 4 und an der darin befindlichen Zellkultur 12 angeordnet werden kann. Auf diese Weise können beispielsweise auch optische Abbildungen mit einer 100-fachen Vergrößerung aufgenommen werden, während eine Krafteinwirkung auf die Zellkultur 12 ausgeübt wird.

Eine vergleichbare Versuchsanordnung kann für die Verwendung mit Mikrotiterplatten vorgesehen und angepasst sein, die über gegebenenfalls wesentlich mehr Kavitäten verfügt, beispielsweise 48, 96 oder 384 sowie mehr oder weniger Kavitäten.

In den Fig. 5 und 6 sind exemplarisch zwei Seitenansichten einer Trägerstruktur 4 dargestellt, die von einem ersten Aktor 5, einem zweiten Aktor 6 und einem dritten Aktor 7 gehalten wird. Die drei Aktoren 5, 6 und 7 bilden die Krafteinwirkungseinrichtung für diese Trägerstruktur 4. Durch die nicht parallel zueinander, sondern in einem Winkel relativ zueinander ausgerichteten Aktoren 5 und 6 kann die Trägerstruktur 4 ergriffen und positioniert werden. Der in den Fig. 5 und 6 jeweils oben dargestellte Aktor 7 kann gegebenenfalls eine Krafteinwirkung erzeugen oder verstärken, die in einer in den Fig. 5 und 6 als Z-Achse bezeichneten Richtung auf die Trägerstruktur 4 ausgeübt wird. Eine derartige Krafteinwirkungseinrichtung kann in jeder Kavität 17 der Versuchsanordnung 15 verwirklicht werden. Insbesondere die Aktoren 7 können verlagerbar an dem Haltegestell 2 gelagert sein, um bei Bedarf und beispielsweise bei optischen Untersuchungen durch das Objektiv 19 aus der taschenförmigen Ausnehmung 13 der Trägerstruktur 4 herausgezogen zu werden.

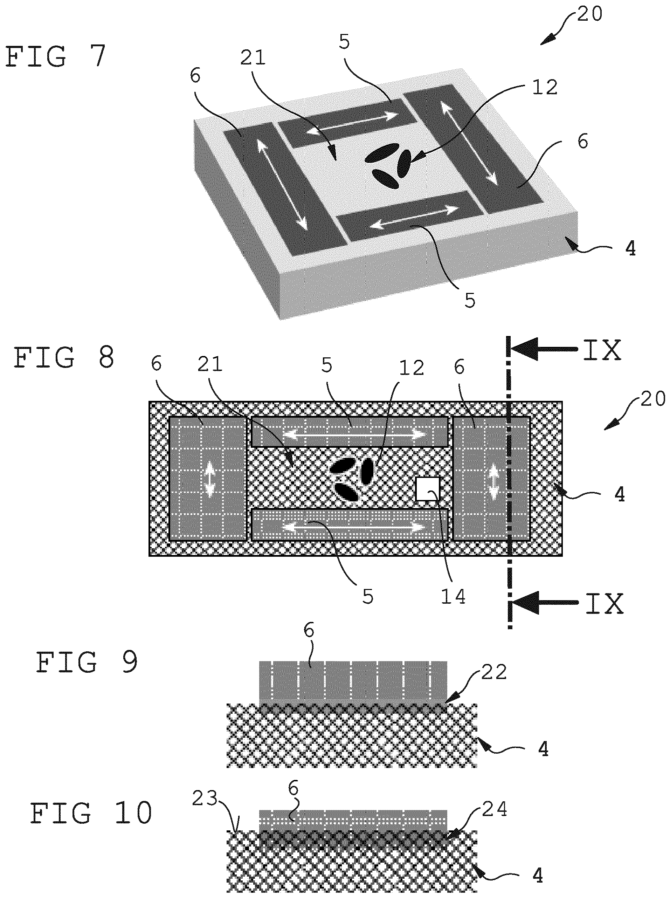

In Fig. 7 ist schematisch eine Ansicht einer wiederum abweichend ausgestalteten Versuchsanordnung 20 mit einer flach und quaderförmig ausgebildeten Trägerstruktur 4 dargestellt, auf der zwei erste Aktoren 5 und zwei zweite Aktoren 6 angeordnet sind. Die beiden ersten Aktoren 5 und die beiden zweiten Aktoren 6 sind jeweils flache streifenförmige elektroaktive Polymerschichtaktoren. Durch eine sich verändernde Spannung kann eine Ausdehnung oder Kontraktion der elektroaktiven Polymerschicht der Aktoren 5, 6 erzwungen werden, die vor allem eine Längenänderung in Längsrichtung der Aktoren 5, 6 bewirkt. Durch eine zeitlich und räumlich koordinierte Betätigung der beiden ersten Aktoren 5 und der beiden zweiten Aktoren 6 kann in einem von den Aktoren 5, 6 eingerahmten Bereich 21 der Trägerstruktur 4 eine kontrollierbare Verformung bewirkt werden. In diesem eingerahmten Bereich 21 ist die Zellkultur 12 angeordnet. Zusätzlich ist in dem eingerahmten Bereich 21 die Sensoreinrichtung 14 eingebettet, mit der die von den Aktoren 5 und 6 auf die Trägerstruktur 4 ausgeübte Krafteinwirkung erfasst werden kann.

Die Aktoren 5 und 6 können beispielsweise mit einer haftvermittelnden Schicht 22 auf die Trägerstruktur 4 aufgeklebt sein, wie es in Fig. 9 schematisch dargestellt ist. Es ist auch möglich, dass bei einer dreidimensional strukturierten Oberfläche 23 der Trägerstruktur 4 die Aktoren 5 und 6 in einen oberflächennahen Bereich 24 der Trägerstruktur 4 eindringen und dadurch formschlüssig auf der Oberfläche 23 der Trägerstruktur 4 festgelegt sind, wie es in Fig. 10 angedeutet ist. Die Trägerstruktur 4 kann auch eine beispielsweise streifenförmige Ausnehmung aufweisen, in welche der zugeordnete Aktor 5, 6 teilweise oder vollständig eingebettet ist. Es ist auch denkbar, dass die Aktoren 5 und 6 aus einem Material bestehen oder mit einem Material überzogen sind, welches eine ausreichend kraftübertragende Haftverbindung mit der Trägerstruktur 4 eingeht, so dass der Aktor 5, 6 nicht in die Trägerstruktur 4 eindringen muss.

## Patentansprüche

1. Versuchsanordnung (1, 15, 20) zum Untersuchen einer Zellkultur (12) unter dynamischer Krafteinwirkung mit einer Trägerstruktur (4) zur Aufnahme der Zellkultur (12) und mit einer Krafteinwirkungsvorrichtung, wobei die Trägerstruktur (4) dreidimensional ausgebildet und so ausgestaltet ist, dass die Zellkultur (12) in die Trägerstruktur (4) eingebettet ist, wobei durch eine Krafteinwirkung eine Verformung der Trägerstruktur (4) bewirkt werden kann, und dass die Krafteinwirkungsvorrichtung einen ersten Aktor (5) aufweist, der beabstandet von der in der Trägerstruktur (4) eingebetteten Zellkultur (12) angeordnet und so ausgerichtet ist, dass der erste Aktor eine gerichtete Kraftwirkung auf die Trägerstruktur (4) ausüben kann, **dadurch gekennzeichnet, dass** die Krafteinwirkungsvorrichtung einen zweiten Aktor (6) aufweist, der beabstandet von der in der Trägerstruktur (4) eingebetteten Zellkultur (12) angeordnet und so ausgerichtet ist, dass der erste Aktor (5) und der zweite Aktor (6) eine voneinander abweichend gerichtete Krafteinwirkung auf die Trägerstruktur (4) ausüben können, und dass die Krafteinwirkungsvorrichtung einen dritten Aktor (7) aufweist, der beabstandet von der in der Trägerstruktur (4) eingebetteten Zellkultur (12) angeordnet und so ausgerichtet ist, dass der dritte Aktor (7) eine von dem ersten Aktor (5) und von dem zweiten Aktor (6) abweichend gerichtete Krafteinwirkung auf die Trägerstruktur (4) ausüben kann, sodass mit Hilfe der drei Aktoren eine resultieren Gesamtkraft in allen Raumrichtungen vorgegeben und ausgeübt werden kann.

2. Versuchsanordnung (1, 15) nach einem der vorausgehenden Ansprüche, **dadurch gekennzeichnet, dass** die Versuchsanordnung (1) ein Haltegestell (2) zur Aufnahme der Trägerstruktur (4) aufweist und dass mindestens einer der Aktoren (5, 6, 7) derart an dem Haltegestell (2) festgelegt ist, dass der Aktor (5, 6, 7) eine die Trägerstruktur (4) verformende Krafteinwirkung auf die in dem Haltegestell (2) aufgenommene Trägerstruktur (4) ausüben kann.

3. Versuchsanordnung (1) nach Anspruch 2, **dadurch gekennzeichnet, dass** der an dem Haltegestell (2) festgelegte mindestens eine Aktor (5, 6, 7) mit einer ersten Seite (10) der Trägerstruktur (4) in Wirkverbindung steht und dass die Trägerstruktur (4) an einer der ersten Seite gegenüberliegenden zweiten Seite mit einer Befestigungseinrichtung an dem Haltegestell (2) festgelegt ist, so dass durch den mindestens einen Aktor (5, 6, 7) Zugkräfte oder Druckkräfte auf die Trägerstruktur (4) übertragen werden können.

4. Versuchsanordnung (1) nach Anspruch 3, **dadurch gekennzeichnet, dass** die Befestigungseinrichtung an der zweiten Seite der Trägerstruktur (4) einen weiteren Gegenaktor (8) aufweist, der an dem Haltegestell (2) festgelegt ist und eine Krafteinwirkung auf die Trägerstruktur (4) ausüben kann.

5. Versuchsanordnung (1, 15) nach einem der voranstehenden Ansprüche 2 bis 4, **dadurch gekennzeichnet, dass** das Haltegestell (2) als Rahmengestell ausgebildet ist und mindestens an zwei gegenüberliegenden Gestellseiten durchsichtige Öffnungen aufweist, durch welche die Trägerstruktur (4) beleuchtet und betrachtet werden kann.

6. Versuchsanordnung (1, 15) nach einem der vorangehenden Ansprüche, **dadurch gekennzeichnet, dass** der erste Aktor (5) und gegebenenfalls weitere Aktoren (6, 7) und/oder Gegenaktoren (8) verlagerbar an dem Rahmengestell gelagert sind.

7. Versuchsanordnung (1, 15, 20) nach einem der vorangehenden Ansprüche, **dadurch gekennzeichnet, dass** die Trägerstruktur (4) mindestens eine Ausnehmung (13) aufweist, in welche der erste Aktor (5) eingreifen kann, um durch eine Krafteinwirkung die Verformung der Trägerstruktur (4) zu bewirken.

8. Versuchsanordnung (1, 15) nach Anspruch 7, **dadurch gekennzeichnet, dass** die Trägerstruktur (4) mindestens zwei Ausnehmungen (13) aufweist, die in einem Winkel zueinander beabstandet zueinander so angeordnet sind, dass ein erster Aktor (5) und ein zweiter Aktor (6) in jeweils eine zugeordnete Ausnehmung eingreifen können, um die Trägerstruktur (4) ergreifen und von einem Untergrund abheben zu können.

9. Versuchsanordnung (1, 15) nach Anspruch 7 oder 8, **dadurch gekennzeichnet, dass** die mindestens eine Ausnehmung (13) in der Trägerstruktur (4) taschenförmig ausgestaltet ist.

10. Versuchsanordnung (20) nach einem der vorangehenden Ansprüche, **dadurch gekennzeichnet, dass** mindestens ein Aktor (5, 6) in einem Abstand von der Zellkultur (12) ausschließlich auf der Trägerstruktur (4) so angeordnet und festgelegt ist, dass durch eine Betätigung des Aktors (5, 6) eine Verformung der Trägerstruktur (4) bewirkt wird.

11. Versuchsanordnung (20) nach Anspruch 10, **dadurch gekennzeichnet, dass** mehrere Aktoren (5, 6) rahmenförmig die Zellkultur (12) umgebend auf einer Oberfläche (23) der Trägerstruktur (4) angeordnet sind.

12. Versuchsanordnung (1, 15, 20) nach einem der vorangehenden Ansprüche, **dadurch gekennzeichnet, dass** die Trägerstruktur (4) Fasern aufweist.

13. Versuchsanordnung (1, 15, 20) nach einem der vorangehenden Ansprüche, **dadurch gekennzeichnet, dass** in die Trägerstruktur (4) ein biokompatibles Hydrogel zur Aufnahme der Zellkultur (12) eingebettet ist oder dass die Trägerstruktur (4) aus einem biokompatiblen Hydrogel besteht.

14. Versuchsanordnung (1, 15, 20) nach einem der vorangehenden Ansprüche, **dadurch gekennzeichnet, dass** mindestens einer der Aktoren (5, 6, 7) und/oder der Gegenaktoren (8) ein Piezoaktor, ein elektrothermischer oder elektroaktiver Polymeraktor oder ein elektromechanischer, elektrochemischer, magnetostriktiver, hydraulischer, pneumatischer, Bi-Metall- oder elektromagnetischer Aktor ist.

15. Versuchsanordnung (1, 15, 20) nach einem der vorangehenden Ansprüche, **dadurch gekennzeichnet, dass** mindestens einer der Aktoren (5, 6, 7) und/oder der Gegenaktoren (8) einen Formgedächtniswerkstoff aufweist.

16. Versuchsanordnung (1, 15, 20) nach einem der vorangehenden Ansprüche, **dadurch gekennzeichnet, dass** in mindestens einem Aktor (5, 6, 7) und/oder in mindestens einem Gegenaktor (8) und/oder in der Trägerstruktur (4) mindestens eine Sensoreinrichtung (14) zur Erfassung der auf die Trägerstruktur (4) einwirkenden Kräfte angeordnet ist.

17. Versuchsanordnung (1, 15) nach einem der voranstehenden Ansprüche 2 bis 16, **dadurch gekennzeichnet, dass** das Haltegestell (2) mehrere Krafteinwirkungseinrichtungen mit jeweils mindestens einem ersten Aktor (5) und mehrere Trägerstrukturen (4) aufweist, wobei mit den mehreren ersten Aktoren (5) jeweils eine die mindestens eine zugeordnete Trägerstruktur (4) verformende Krafteinwirkung auf die betreffende Trägerstruktur (4) ausgeübt wird.

18. Versuchsanordnung (1) mit einer Trägerstruktur (4) zur Aufnahme der Zellkultur (12) und mit einer Krafteinwirkungsvorrichtung, wobei die Trägerstruktur (4) dreidimensional ausgebildet und so ausgestaltet ist, dass die Zellkultur (12) in die Trägerstruktur (4) eingebettet ist, wobei durch eine Krafteinwirkung eine Verformung der Trägerstruktur (4) bewirkt werden kann, und dass die Krafteinwirkungsvorrichtung einen ersten Aktor (5) aufweist, der beabstandet von der in der Trägerstruktur (4) eingebetteten Zellkultur (12) angeordnet und so ausgerichtet ist, dass der erste Aktor eine gerichtete Kraftwirkung auf die Trägerstruktur (4) ausüben kann, nach Anspruch 17 und wobei die Versuchsanordnung ein Haltegestell (2) mit mehreren Krafteinwirkungseinrichtungen mit jeweils mindestens einem ersten Aktor (5) und mehrere Trägerstrukturen (4) aufweist, sodass mit den mehreren ersten Aktoren (5) jeweils eine die mindestens eine zugeordnete Trägerstruktur (4) verformende Krafteinwirkung auf die betreffende Trägerstruktur (4) ausgeübt werden kann, **dadurch gekennzeichnet, dass** das Haltegestell (2) eine Verbindungseinrichtung zur Verbindung mit einer Mikrotiterplatte (16) aufweist, so dass das Haltegestell (2) derart mit der Mikrotiterplatte (16) verbunden werden kann, dass jede Trägerstruktur (4) in einer zugeordneten Kavität (17) der Mikrotiterplatte (16) angeordnet und mit dem zugeordneten ersten Aktor (5) verformt werden kann.

## Claims

1. Testing arrangement (1, 15, 20) for examining a cell culture (12) under the effect of a dynamic force, comprising a carrier structure (4) for receiving the cell culture (12) and comprising a force-application device, wherein the carrier structure (4) is three-dimensional and designed such that the cell culture (12) is embedded in the carrier structure (4), wherein it is possible to bring about deformation of the carrier structure (4) by means of a force application, and that the force-application device comprises a first actuator (5) which is arranged spaced apart from the cell culture (12) embedded in the carrier structure (4) and is oriented such that the first actuator can apply force to the carrier structure (4) in a directed manner, **characterised in that** the force-application device comprises a second actuator (6), which is arranged spaced apart from the cell culture (12) embedded in the carrier structure (4) and is oriented such that the first actuator (5) and the second actuator (6) can apply forces in different directions to the carrier structure (4), and **in that** the force-application device comprises a third actuator (7), which is arranged spaced apart from the cell culture (12) embedded in the carrier structure (4) and is oriented such that the third actuator (7) can apply a force to the carrier structure (4) in a direction different from those of the first actuator (5) and the second actuator (6), such that, by means of the three actuators, a resulting total force can be predetermined and exerted in all spatial directions.

2. Testing arrangement (1, 15) according to the preceding claim, **characterised in that** the testing arrangement (1) comprises a supporting frame (2) for receiving the carrier structure (4), and **in that** at least one of the actuators (5, 6, 7) is secured to the supporting frame (2) such that the actuator (5, 6, 7) can apply a force that deforms the carrier structure (4) to the carrier structure (4) received in the supporting frame (2).

3. Testing arrangement (1) according to claim 2, **characterised in that** the at least one actuator (5, 6, 7) fixed to the supporting frame (2) is operatively connected to a first side (10) of the carrier structure (4), and **in that** the carrier structure (4) is secured to the supporting frame (2) by a fastening means on a second side opposite the first side, such that, by means of the at least one actuator (5, 6, 7), tensile forces or compressive forces can be transferred to the carrier structure (4).

4. Testing arrangement (1) according to claim 3, **characterised in that**, on the second side of the carrier structure (4), the fastening means comprises an additional counter-actuator (8), which is secured to the supporting frame (2) and can apply a force to the carrier structure (4).

5. Testing arrangement (1, 15) according to any of the preceding claims 2 to 4, **characterised in that** the supporting frame (2) is designed as a rack and, at least on two opposite rack sides, comprises transparent openings through which the carrier structure (4) can be illuminated and viewed.

6. Testing arrangement (1, 15) according to any of the preceding claims, **characterised in that** the first actuator (5) and optionally additional actuators (6, 7) and/or counter-actuators (8) are mounted on the rack so as to be displaceable.

7. Testing arrangement (1, 15, 20) according to any of the preceding claims, **characterised in that** the carrier structure (4) comprises at least one recess (13), in which the first actuator (5) can engage in order to bring about deformation of the carrier structure (4) by force application.

8. Testing arrangement (1, 15) according to claim 7, **characterised in that** the carrier structure (4) comprises at least two recesses (13), which are arranged at an angle to one another and so as to be spaced apart from one another, such that a first actuator (5) and a second actuator (6) can each engage in an associated recess in order to be able to grasp the carrier structure (4) and lift it from a surface.

9. Testing arrangement (1, 15) according to claim 7 or 8, **characterised in that** the at least one recess (13) in the carrier structure (4) is pocket-shaped.

10. Testing arrangement (20) according to any of the preceding claims, **characterised in that** at least one actuator (5, 6) is solely arranged on and secured to the carrier structure (4) at a distance from the cell culture (12) such that the carrier structure (4) is deformed by actuating the actuator (5, 6).

11. Testing arrangement (20) according to claim 10, **characterised in that** a plurality of actuators (5, 6) are arranged on a surface (23) of the carrier structure (4) so as to surround the cell culture (12) in a frame shape.

12. Testing arrangement (1, 15, 20) according to any of the preceding claims, **characterised in that** the carrier structure (4) comprises fibres.

13. Testing arrangement (1, 15, 20) according to any of the preceding claims, **characterised in that** a biocompatible hydrogel is embedded in the carrier structure (4) for receiving the cell culture (12), or **in that** the carrier structure (4) consists of a biocompatible hydrogel.

14. Testing arrangement (1, 15, 20) according to any of the preceding claims, **characterised in that** at least one of the actuators (5, 6, 7) and/or the counter-actuators (8) is a piezo actuator, an electrothermal or electroactive polymer actuator or an electromechanical, electrochemical, magnetostrictive, hydraulic, pneumatic, bimetal or electromagnetic actuator.

15. Testing arrangement (1, 15, 20) according to any of the preceding claims, **characterised in that** at least one of the actuators (5, 6, 7) and/or the counter-actuators (8) comprises a shape-memory material.

16. Testing arrangement (1, 15, 20) according to any of the preceding claims, **characterised in that** at least one sensor apparatus (14) for detecting the forces acting on the carrier structure (4) is arranged in at least one actuator (5, 6, 7) and/or in at least one counter-actuator (8) and/or in the carrier structure (4).

17. Testing arrangement (1, 15) according to any of the preceding claims 2 to 16, **characterised in that** the supporting frame (2) comprises a plurality of force-application apparatuses with in each case at least one actuator (5) and a plurality of carrier structures (4), wherein the plurality of first actuators (5) each apply a force that deforms the at least one associated carrier structure (4) to the relevant carrier structure (4).

18. Testing arrangement (1) comprising a carrier structure (4) for receiving the cell culture (12) and comprising a force-application device, wherein the carrier structure (4) is three-dimensional and is designed such that the cell culture (12) is embedded in the carrier structure (4), wherein it is possible to bring about deformation of the carrier structure (4) by force application, and that the force-application device comprises a first actuator (5), which is spaced apart from the cell culture (12) embedded in the carrier structure (4) and is oriented such that the first actuator can apply a force to the carrier structure (4) in a directed manner, according to claim 17, and wherein the testing arrangement comprises a supporting frame (2) with a plurality of force-application apparatuses with in each case at least one first actuator (5) and a plurality of carrier structures (4), such that, by means of the plurality of first actuators (5), a force that deforms the at least one associated carrier structure (4) can be applied to the relevant carrier structure (4), **characterised in that** the supporting frame (2) comprises a connecting means for connection to a microtiter plate (16), such that the supporting frame (2) can be connected to the microtiter plate (16) in such a way that each carrier structure (4) is arranged in an associated cavity (17) of the microtiter plate (16) and can be deformed by the associated first actuator (5).

## Revendications

1. Agencement expérimental (1, 15, 20) servant à analyser une culture de cellules (12) sous l'application d'une force dynamique avec une structure de support (4) servant à recevoir la culture de cellules (12) et avec un dispositif d'application de force, dans lequel la structure de support (4) est réalisée de manière tridimensionnelle et est configurée de telle sorte que la culture de cellules (12) est encastrée dans la structure de support (4), dans lequel une déformation de la structure de support (4) peut être provoquée par une application de force, et le dispositif d'application de force présente un premier actionneur (5), qui est disposé à distance de la culture de cellules (12) encastrée dans la structure de support (4) et est orienté de telle sorte que le premier actionneur peut exercer une application de force dirigée sur la structure de support (4),
**caractérisé en ce que** le dispositif d'application de force présente un deuxième actionneur (6), qui est disposé à distance de la culture de cellules (12) encastrée dans la structure de support (4) et est orienté de telle sorte que le premier actionneur (5) et le deuxième actionneur (6) peuvent exercer une application de force dirigée de manière différente l'une de l'autre sur la structure de support (4), et que le dispositif d'application de force présente un troisième actionneur (7), qui est disposé à distance de la culture de cellules (12) encastrée dans la structure de support (4) et est orienté de telle sorte que le troisième actionneur (7) peut exercer une application de force dirigée de manière différente de celles du premier actionneur (5) et du deuxième actionneur (6) sur la structure de support (4) de sorte qu'à l'aide des trois actionneurs, une force globale résultante peut être spécifiée et exercée dans toutes les directions spatiales.

2. Agencement expérimental (1, 15) selon la revendication précédente, **caractérisé en ce que** l'agencement expérimental (1) présente un châssis de maintien (2) servant à recevoir la structure de support (4), et qu'au moins un des actionneurs (5, 6, 7) est immobilisé de telle manière au niveau du châssis de maintien (2) que l'actionneur (5, 6, 7) peut exercer sur la structure de support (4) reçue dans le châssis de maintien (2) une application de force déformant la structure de support (4) .

3. Agencement expérimental (1) selon la revendication 2, **caractérisé en ce que** l'au moins un actionneur (5, 6, 7) immobilisé au niveau du châssis de maintien (2) est en liaison fonctionnelle avec un premier côté (10) de la structure de support (4), et que la structure de support (4) est immobilisée au niveau du châssis de maintien (2) avec un moyen de fixation au niveau d'un deuxième côté faisant face au premier côté de sorte que des forces de traction ou des forces de pression peuvent être transmises sur la structure de support (4) par l'au moins un actionneur (5, 6, 7).

4. Agencement expérimental (1) selon la revendication 3, **caractérisé en ce que** le moyen de fixation présente au niveau du deuxième côté de la structure de support (4) un autre contre-actionneur (8), qui est immobilisé au niveau du châssis de maintien (2) et peut exercer une application de force sur la structure de support (4).

5. Agencement expérimental (1, 15) selon l'une quelconque des revendications précédentes 2 à 4, **caractérisé en ce que** le châssis de maintien (2) est réalisé en tant que châssis de cadre et présente au moins au niveau de deux côtés de châssis se faisant face des ouvertures transparentes, à travers lesquelles la structure de support (4) peut être éclairée et observée.

6. Agencement expérimental (1, 15) selon l'une quelconque des revendications précédentes, **caractérisé en ce que** le premier actionneur (5) et éventuellement d'autres actionneurs (6, 7) et/ou contre-actionneurs (8) sont montés de manière à pouvoir être déplacés au niveau du châssis de cadre.

7. Agencement expérimental (1, 15, 20) selon l'une quelconque des revendications précédentes, **caractérisé en ce que** la structure de support (4) présente au moins un évidement (13), avec lequel le premier actionneur (5) peut venir en prise pour provoquer par une application de force la déformation de la structure de support (4).

8. Agencement expérimental (1, 15) selon la revendication 7, **caractérisé en ce que** la structure de support (4) présente au moins deux évidements (13), qui sont disposés l'un par rapport à l'autre à distance selon un angle l'un par rapport à l'autre de telle sorte qu'un premier actionneur (5) et un deuxième actionneur (6) peuvent venir en prise avec respectivement un évidement associé pour pouvoir saisir la structure de support (4) et la soulever d'un sol.

9. Agencement expérimental (1, 15) selon la revendication 7 ou 8, **caractérisé en ce que** l'au moins un évidement (13) est réalisé en forme de poche dans la structure de support (4) .

10. Agencement expérimental (20) selon l'une quelconque des revendications précédentes, **caractérisé en ce qu'**au moins un actionneur (5, 6) est disposé et immobilisé exclusivement sur la structure de support (4) à une distance de la culture de cellules (12) de telle sorte qu'une déformation de la structure de support (4) est provoquée par un actionnement de l'actionneur (5, 6).

11. Agencement expérimental (20) selon la revendication 10, **caractérisé en ce que** plusieurs actionneurs (5, 6) sont disposés sur une surface (23) de la structure de support (4) de manière à entourer la culture de cellules (12) en forme de cadre.

12. Agencement expérimental (1, 15, 20) selon l'une quelconque des revendications précédentes, **caractérisé en ce que** la structure de support (4) présente des fibres.

13. Agencement expérimental (1, 15, 20) selon l'une quelconque des revendications précédentes, **caractérisé en ce qu'**un hydrogel biocompatible servant à recevoir la culture de cellules (12) est encastrée dans la structure de support (4), ou que la structure de support (4) consiste en un hydrogel biocompatible.

14. Agencement expérimental (1, 15, 20) selon l'une quelconque des revendications précédentes, **caractérisé en ce qu'**au moins un des actionneurs (5, 6, 7) et/ou des contre-actionneurs (8) est un actionneur piézoélectrique, un actionneur polymère électrothermique ou électroactif ou un actionneur électromécanique, électrochimique, magnétostrictif, hydraulique, pneumatique, bimétal ou électromagnétique.

15. Agencement expérimental (1, 15, 20) selon l'une quelconque des revendications précédentes, **caractérisé en ce qu'**au moins un des actionneurs (5, 6, 7) et/ou des contre-actionneurs (8) présente un matériau à mémoire de forme.

16. Agencement expérimental (1, 15, 20) selon l'une quelconque des revendications précédentes, **caractérisé en ce qu'**au moins un moyen de capteur (14) servant à détecter les forces agissant sur la structure de support (4) est disposé dans au moins un actionneur (5, 6, 7) et/ou dans au moins un contre-actionneur (8) et/ou dans la structure de support (4).

17. Agencement expérimental (1, 15) selon l'une quelconque des revendications précédentes 2 à 16, **caractérisé en ce que** le châssis de maintien (2) présente plusieurs moyens d'application de force avec respectivement au moins un premier actionneur (5) et des plusieurs structures de support (4), dans lequel respectivement une application de force, déformant l'au moins une structure de support (4) associée, est exercée sur la structure de support (4) concernée avec les plusieurs premiers actionneurs (5).

18. Agencement expérimental (1) avec une structure de support (4) servant à recevoir la culture de cellules (12) et avec un dispositif d'application de force, dans lequel la structure de support (4) est réalisée de manière tridimensionnelle et est configurée de telle sorte que la culture de cellules (12) est encastrée dans la structure de support (4), dans lequel une déformation de la structure de support (4) peut être provoquée par une application de force, et que le dispositif d'application de force présente un premier actionneur (5), qui est disposé à distance de la culture de cellules (12) encastrée dans la structure de support (4) et est orienté de telle sorte que le premier actionneur peut exercer une application de force dirigée sur la structure de support (4), selon la revendication 17, et dans lequel l'agencement expérimental présente un châssis de maintien (2) avec plusieurs moyens d'application de force avec respectivement au moins un premier actionneur (5) et plusieurs structures de support (4) de sorte que respectivement une application de force déformant l'au moins une structure de support (4) associée peut être exercée sur la structure de support (4) concernée avec les plusieurs premiers actionneurs (5), **caractérisé en ce que** le châssis de maintien (2) présente un moyen de liaison destiné à être relié à une plaque de microtitrage (16) de sorte que le châssis de maintien (2) peut être relié de telle manière à la plaque de microtitrage (16) que chaque structure de support (4) peut être disposée dans une cavité (17) associée de la plaque de microtitrage (16) et déformée avec le premier actionneur (5) associé.
